# EUROPÄISCHE PATENTANMELDUNG

(11) **EP 4 241 746 A1**
(43) Veröffentlichungstag der Anmeldung: **13.09.2023**
(21) Anmeldenummer: 23160218.6
(22) Anmeldetag: 06.03.2023
(51) Int. Cl.: A61F 7/00, H02H 3/16, H02J 3/14

(54) **HYPOTHERMIEGERÄT UND VERFAHREN ZUM BETREIBEN EINES HYPOTHERMIEGERÄTES**

(30) Priorität: 11.03.2022 DE 102022105796
(71) Anmelder: Lauda Dr. R. Wobser GmbH & Co. KG, 97922 Lauda-Königshofen (DE)
(72) Erfinder: Barthel, Georg, 97947 Grünsfeld (DE)
(74) Vertreter: Zimmermann & Partner Patentanwälte mbB

(57) **Zusammenfassung**

Hypothermiegerät mit: einer Mehrzahl von Verbrauchern, welche im Betrieb elektrische Energie verbrauchen; einem Netzanschluss zur Versorgung der Verbraucher mit elektrischer Energie über zumindest zwei Verteiler zum Verteilen der elektrischen Energie ; und einer Mehrzahl von Schaltern, wobei mindestens einer der Verbraucher mittels mindestens eines ihm zugeordneten Schalters (31, 32, 33, 34) mit den Verteilern verbindbar sind; wobei die Schalter (31, 32, 33, 34) derart ausgeführt sind, so dass die Verbraucher allpolig gegenüber den Verteilern schaltbar sind.

## Beschreibung

### Gebiet der Erfindung

Die Erfindung betrifft ein Hypothermiegerät und ein Verfahren zum Betreiben eines Hypothermiegerätes.

### Stand der Technik

Aus dem Stand der Technik sind Hypothermiegeräte bekannt, welche dazu vorgesehen sind, über das Blut den Körper eines Menschen oder eines Säugetiers temporär zu kühlen oder wieder aufzuheizen. Weiterhin sind Apparate bekannt, die lediglich eine Heizleistung bereitstellen. Beispielhaft sei übergreifend auf die US 6,423,268 verwiesen.

Bei vielen Herzoperationen kommt eine Herz-Lungen-Maschine zum Einsatz. Die Herz-Lungen-Maschine übernimmt für die Dauer des chirurgischen Eingriffs die Pumpfunktion des Herzens sowie die Oxygenierung und Kohlendioxid-Elimination des Blutes. Durch zusätzliche Kühlung des Blutes und damit der Körpertemperatur kann die vom Patienten tolerierte Operationszeit wesentlich verlängert werden. Nach dem Eingriff müssen das Blut und damit der Patient wieder auf 37 °C erwärmt werden. Diese Temperierung wird durch das Hypothermiegerät ermöglicht, das über einen Wärmeträgerkreislauf, beispielsweise mit Wasser, mittels Wärmetauscher das Blut heizt und kühlt und so den Patienten temperiert.

Darüber hinaus kann es in dem Hypothermiegerät noch einen zweiten Kreislauf für die Kardioplegie geben. Hier wird ebenfalls über einen Wärmetauscher Kardioplegielösung temperiert, die dann zyklisch dem Herzmuskel zugeführt wird, um dessen Aktivität während der Operation zu unterbinden.

Fließt ein Ableitstrom im Fehlerfall als erhöhter Berührungsstrom durch den Anwender des Gerätes oder durch eine andere Person, so kann der Ableitstrom einen elektrischen Schlag auslösen. Ein zu betrachtender Fehler als Ursache für einen solchen Ableitstrom ist der Verlust der Erdung des Geräts bei PE-Anschluss über die Netzleitung. Ein alleine auftretender Fehler wird auch als so genannter erster Fehler bezeichnet.

Der Berührungsstrom darf im Normalzustand, also bei Betrieb des Gerätes ohne eine Beschädigung einer Sicherungseinrichtung 0,1 mA nicht übersteigen. Bei einem ersten Fehler darf der Berührungsstrom 0,5 mA nicht überschreiten. In Europa ist dies beispielsweise in der Norm EN 60601-1 gefordert. Dort oder weiteren, vergleichbaren Regelwerken können auch Beispiele erster Fehler entnommen werden.

Bei Hypothermiegeräten können verschiedene, mit Netzspannung betriebene Verbraucher einen Ableitstrom erzeugen oder zu einem Ableitstrom beitragen. Mit immer leistungsfähigeren Hypothermiegeräten kann es schwierig sein, die Grenzen für den Ableitstrom einzuhalten.

### Offenbarung der Erfindung

Aufgabe der Erfindung ist es, ein Hypothermiegerät und ein Verfahren zum Betreiben eines gegenüber dem Stand der Technik verbesserten Hypothermiegeräts anzugeben, insbesondere soll die Sicherheit des Gerätes während des Betriebs erhöht werden.

Die Aufgabe wird z. B. gelöst durch ein Hypothermiegerät gemäß Anspruch 1 und durch ein Verfahren gemäß dem nebengeordneten Anspruch. Weiterbildungen sind in den abhängigen Ansprüchen angegeben.

Bei Ausführungsformen der Erfindung ist eine Mehrzahl von Verbrauchern vorgesehen, welche im Betrieb elektrische Energie verbrauchen. Diese Verbraucher werden mittels eines Netzanschlusses mit elektrischer Energie über zumindest zwei Verteiler zum Verteilen der elektrischen Energie versorgt. Weiterhin ist eine Mehrzahl von Schaltern vorgesehen, wobei mindestens einer, typischerweise zwei oder mehr, vorzugsweise die meisten der Verbraucher mittels eines ihm zugeordneten Schalters mit den Verteilern verbindbar ist.

Die Verteiler zum Verteilen der elektrischen Energie können beispielsweise als Sammelschiene ausgeführt sein, wobei unter Sammelschiene jedwede Art von Anordnung von Leitern, beispielsweise Kabeln, Schienen, oder Leitern einer Leiterplatte, zum Verteilen der elektrischen Energie verstanden wird.

Die Verbraucher sind typischerweise unabhängig voneinander oder in Gruppen verbindbar. Bei Ausführungsformen können auch so genannte Klein-Verbraucher vorgesehen sein, welche während des Betriebs des Hypothermiegerätes dauerhaft mit dem Netzanschluss verbunden sind, bspw. eine Kontrolleinrichtung des Hypothermiegerätes. Die Kontrolleinrichtung wird typischerweise benötigt und ist dazu eingerichtet, die typischen Verfahrensschritte der hierin beschriebenen Verfahren zu steuern und zu kontrollieren.

Die Erfindung sieht vor, dass die Schalter derart ausgeführt sind, dass die Verbraucher allpolig gegenüber den Verteilern schaltbar sind. Dies bietet gegenüber einer Trennung nur eines Pols oder einer Teilmenge der Pole, über welche der Verbraucher mit elektrischer Energie versorgt werden kann, den Vorteil, dass jeglicher Stromfluss von oder zu dem jeweiligen Verbraucher unterbunden wird.

Die Schalter können einpolig oder mehrpolig ausgeführt sein. Beispielsweise kann pro Verbraucher jeweils ein einpoliger Schalter zu jedem der Verteiler vorgesehen sein. Zum allpoligen Schalten eines Verbrauchers gegenüber den Verteilern können die diesem Verbraucher zugeordneten Schalter bei typischen Ausführungsformen synchron geschaltet werden.

Bei weiteren typischen Ausführungsformen sind die Schalter mehrpolig, insbesondere entsprechend der Anzahl der Verteiler, ausgeführt. So können beispielsweise zweipolige Schalter vorgesehen sein bei einem Hypothermiegerät, welches zwei Verteiler aufweist. Typischerweise können Ausführungsformen auch jeweils einpolige und mehrpolige Schalter für verschiedene Verbraucher aufweisen.

Typische Verfahren zum Betreiben von Hypothermiegeräten werden insbesondere mit den hierin beschriebenen Ausführungsformen von Hypothermiegeräten durchgeführt.

Der Beitrag zum Ableitstrom eines einzelnen Verbrauchers wird durch allpoliges Trennen dieses Verbrauchers vom Netz unterbunden. Auf diese Weise kann der Grenzwert auch mit Hypothermiegeräten mit leistungsfähigen Heizungen und Kältemaschinen zumindest dann eingehalten werden, wenn nicht alle Verbraucher durch das allpolige Trennen mit dem Netzanschluss verbunden sind.

Typische Hypothermiegeräte umfassen eine Kontrolleinrichtung, welche dazu eingerichtet ist, insbesondere in einem Patienten-Betrieb zumindest einen der Verbraucher mittels des diesem Verbraucher zugeordneten mindestens einen Schalters allpolig von den Verteilern zu trennen.

Typische Vorteile von Ausführungsformen sind dazu eingerichtet, zur Temperierung eines Patienten eingesetzt zu werden. Typischerweise ist mit dem Begriff "Betrieb" hierin ein Patienten-Betrieb, d. h. ein Betrieb mit Temperierung eines Patienten oder eines Säugetiers gemeint. Typische Ausführungsformen sind dazu eingerichtet, beispielsweise über eine so genannte Herz-Lungen-Maschine im Betrieb einen Patienten zu temperieren.

Bei typischen Ausführungsformen ist die Kontrolleinrichtung dazu eingerichtet, so viele der Verbraucher mittels der diesen Verbrauchern jeweils zugeordneten Schaltern allpolig von den Verteilern zu trennen, so dass in einem fehlerfreien Zustand des Hypothermiegeräts ein Ableitstrom 0,1 mA und/oder im Fall eines ersten Fehlers ein Ableitstrom von 0,5 mA nicht überschritten wird. Ein typischer erster Fehler kann beispielsweise ein Erdungsverlust sein. Insbesondere ist die Kontrolleinrichtung dazu eingerichtet, während eines Betriebs des Hypothermiegeräts die Ableitströme einzuhalten.

Typischerweise umfassen die Verbraucher zumindest zwei der folgenden Einrichtungen: eine Hypothermie-Heizung, eine Kardioplegie-Heizung, eine Kältemaschine und ein Netzteil für die Bereitstellung einer DC-Kleinspannung, typischerweise 12 V DC oder 24 V DC. Typischerweise können eine Mehrzahl von Hypothermie-Heizungen und andere zuvor genannte Verbraucher vorgesehen sein, beispielsweise zwei oder drei. Typische Leistungsdaten von Hypothermie-Heizungen können mindestens 1000 W oder eine Stromaufnahme von mehr als 5 A sein. Typischerweise kann eine Mehrzahl von Kardioplegie-Heizungen vorgesehen sein, beispielsweise zwei oder drei. Typische Leistungsdaten von Kardioplegie-Heizungen können mindestens 1000 W oder eine Stromaufnahme von mehr als 5 A sein. Typischerweise kann eine Kältemaschine mit mindestens 1000 W oder einer Stromaufnahme von mehr als 10 A vorgesehen sein. Bei Ausführungsformen ist ein Netzteil vorgesehen, welches beispielsweise eine Leistungsaufnahme von mehr als 200 W oder eine Stromaufnahme von mehr als 1 A aufweist. Typischerweise kann das Netzteil zur Versorgung unter anderem der Kontrolleinrichtung dienen. Bei Ausführungsformen kann ein Abschalten oder allpoliges Trennen des Netzteils nicht vorgesehen sein, um den Betrieb einer Kontrolleinrichtung aufrecht zu erhalten.

Typische Kontrolleinrichtungen umfassen einen Signaleingang und sind dazu eingerichtet, folgende Schritte auszuführen: Auswählen bestimmter Verbraucher in Abhängigkeit von Betriebsparametern des Hypothermiegeräts und/oder in Abhängigkeit einer über den Signaleingang empfangenen Temperierungsanforderung; Überprüfen, ob bei Verbinden der ausgewählten Verbraucher zumindest eine der zuvor festgelegten Anforderungen erfüllt bleibt; und Verbinden der ausgewählten Verbraucher mittels der diesen Verbrauchern zugeordneten Schaltern mit den Verteilern oder allpoliges Trennen der anderen Verbraucher von den Verteilern mittels der diesen anderen Verbrauchern zugeordneten Schaltern. Typischerweise werden Verbraucher, welche ausgewählt sind, allpolig mittels der Schalter mit den Verteilern verbunden.

Typische Hypothermiegeräte sind mit mindestens zwei Verteilern ausgestattet, beispielsweise für einen einphasigen Betrieb mit Wechselstrom. Der Betrieb kann zwischen einem Phasenleiter und Null erfolgen (Phase - Null). Weitere Ausführungsformen sind alternativ oder zusätzlich für einen Betrieb mit Wechselstrom an zwei Phasenleitern, Phase - Phase, ausgelegt. Insbesondere in den USA ist ein Betrieb an zwei Phasenleitern üblich, wobei zwischen den Verteilern dann eine Spannung von nominal 240 V anliegt.

Bei typischen Ausführungsformen umfasst der Schritt des Überprüfens für den Fall, dass die zumindest eine zuvor festgelegte Anforderung mit den zuvor ausgewählten Verbrauchern nicht erfüllt bleibt: Abwählen von bestimmten Verbrauchern an Hand eines abgespeicherten Prioritätsschemas, so dass die zuvor festgelegte Anforderung mit den verbleibenden ausgewählten Verbrauchern erfüllt wird. Dabei wird unter "Abwählen" typischerweise auch verstanden, dass die jeweiligen Verbraucher, insbesondere Heizungen, mit einer geringeren Leistung mittels Halbwellenansteuerung betrieben werden. Die Leistungsreduzierung im Rahmen des Abwählens erfolgt demnach durch getaktetes allpoliges Trennen bzw. Verbinden der Verbraucher, insbesondere Heizungen, von bzw. mit den Verteilern.

Das abgespeicherte Prioritätsschema kann beispielsweise eine Tabelle sein, welche für einige der Verbraucher vor anderen Verbrauchern priorisiert - je nach Betriebszustand des Hypothermiegerätes oder je nach einer Temperierungsanforderung eines an das Hypothermiegerät angeschlossenen Herz-Lungen-Gerätes oder des Bedieners.

Zur Priorisierung können bei Ausführungsformen so genannte Leistungsmanagementsysteme weitergebildet werden, welche die maximale Stromaufnahme eines Gerätes mit einer zusammengenommenen Stromaufnahme von allen potenziellen Verbrauchern des Gerätes von mehr als 16 A an einer gewöhnlichen ein-phasigen mit 16 A abgesicherten Steckdose (1-Phasen Wechselspannung) ermöglichen. Bei weiteren Ausführungsformen kann die entsprechende Grenze 13 A oder 15 A betragen.

Statt einem Abregeln einzelner Verbraucher auf nur einer Zuleitung sieht die Erfindung eine allpolige Trennung vor, um nicht nur die Leistungsaufnahme, sondern auch den Ableitstrom zu reduzieren. Dabei kann die Einhaltung der Grenze des Ableitstroms auch unabhängig oder zusätzlich zu einem Leistungsmanagementsystem erfolgen.

Typischerweise bezeichnet die mindestens eine Anforderung einen Grenzwert für den maximalen Ableitstrom und/oder einen Grenzwert für eine maximale Stromaufnahme der Verbraucher über die Verteiler. Das heißt, dass bei Ausführungsformen die "mindestens eine Anforderung" mindestens eine dieser zwei Anforderungen darstellen kann oder andere oder auch noch weitere Anforderungen.

Bei typischen Ausführungsformen werden in einer abgespeicherten Tabelle Werte für die jeweilige Stromaufnahme jedes Verbrauchers oder Werte für den Ableitstrom jedes Verbrauchers hinterlegt. Die Prüfung, ob eine Anforderung eingehalten wird, kann dann typischerweise an Hand der Tabelle erfolgen. Typischerweise kann mit der Stromaufnahme auch gleichzeitig der Ableitstrom überwacht werden, indem man sich zu Nutze macht, dass bei typischen Verbrauchern der maximale Ableitstrom eines Verbrauchers zumindest im Wesentlichen proportional zur maximalen Leistungsaufnahme dieses Verbrauchers ist. Dadurch kann bei geeigneter Wahl der maximalen Stromaufnahme der mit den Verteilern verbundenen Verbraucher, beispielsweise 16 A, auch gleichzeitig der Ableitstrom überwacht werden.

Bei typischen Ausführungsbeispielen umfassen die Schalter elektronische Schalter oder Relais oder sind als elektronische Schalter oder als Relais ausgeführt. Typischerweise umfassen Ausführungsformen sowohl elektronische Schalter als auch Relais als "Schalter", beispielsweise elektronische Schalter zum Schalten von Heizungen oder Relais zum Schalten einer Kältemaschine, insbesondere eines Verdichters einer Kältemaschine.

Bei typischen Hypothermiegeräten von Ausführungsformen werden als elektronische Schalter TRIAC-Halbleiter verwendet. Durch den Doppelschalter an beiden Polen L und N (TRIAC) tragen nur "aktive" Halbwellen, also Leistung übertragende Halbwellen, zum Ableitstrom bei. In Zeiten, in denen keine Halbwelle durchgeschaltet wird, ist die Heizung allpolig vom Netz getrennt und bewirkt auch keinen Ableitstrom.

Typischerweise wird zumindest ein Teil der Schalter bei Ausführungsformen mittels einer Halbwellenansteuerung angesteuert, insbesondere durch die Kontrolleinrichtung. Typische Kontrolleinrichtungen von Ausführungsformen sind dazu eingerichtet, die Schalter mittels einer Halbwellenansteuerung anzusteuern. Eine solche Halbwellenansteuerung ist beispielhaft in der DE 101 60 912 A beschrieben. Mit einer Halbwellensteuerung kann beispielsweise die maximale Heizleistung einer über den zugeordneten Schalter geschalteten Heizung fein in Netzhalbwellen aufgeteilt werden, beispielsweise in 10 ms-Schritten bei 50 Hz Netzfrequenz. Halbwellen werden typischerweise gemäß dem Verfahren aus Patent DE 101 60 912 und einem übergeordneten Leistungsmanagement auf die Heizungen gegeben.

Eine Kältemaschine oder ein Verdichter einer Kältemaschine wird typischerweise minutenweise zu- und abgeschaltet. Typische Mindest-Intervalle zum Zu- oder Abschalten der Kältemaschine betragen mindestens 20 Sekunden oder mindestens 45 Sekunden oder mindestens 60 Sekunden. Typischerweise beträgt die maximale Dauer des Mindest-Intervalls zum Zu- oder Abschalten der Kältemaschine höchstens 1:30 Minuten oder höchstens 3 Minuten. Typischerweise wird das Halbwellenverfahren bei Kältemaschinen nicht verwendet. Im ausgeschalteten Zustand wird die Kältemaschine damit mittels des Schalters allpolig vom Netz getrennt und trägt so im nicht aktiven Zustand ebenfalls nicht zum Ableitstrom bei.

Typische Ausführungsformen von Verfahren der Erfindung zum Steuern eines Hypothermigerätes umfassen ein Auswählen bestimmter Verbraucher in Abhängigkeit von Betriebsparametern des Hypothermiegeräts und/oder in Abhängigkeit einer über einen Signaleingang empfangenen Temperierungsanforderung oder des Bedieners. Weiterhin wird eine Überprüfung durchgeführt, ob bei Verbinden der ausgewählten Verbraucher zumindest eine zuvor festgelegte Anforderung erfüllt bleibt. Typischerweise erfolgt ein Verbinden der ausgewählten Verbraucher mit den Verteilern mittels der diesen Verbrauchern zugeordneten Schaltern und/oder allpoliges Trennen der anderen Verbraucher von den Verteilern mittels der diesen anderen Verbrauchern zugeordneten Schaltern.

Bei typischen Verfahren umfasst der Schritt des Überprüfens: Falls die zuvor festgelegte Anforderung mit den zuvor ausgewählten Verbrauchern nicht erfüllt bleibt: Abwählen von bestimmten in dem Schritt des Auswählens ausgewählter Verbraucher an Hand eines abgespeicherten Prioritätsschemas, so dass die zuvor festgelegte Anforderung mit den verbleibenden ausgewählten Verbrauchern erfüllt wird.

Typische Vorteile von Ausführungsformen sind ein sicher beherrschbarer Ableitstrom, die Möglichkeit typische Hypothermiegeräte gemäß der Erfindung mit vergleichsweise hohen Kühl- und Heizleistungen an einer einpoligen Steckdose zu betreiben, insbesondere unter Einhaltung der maximalen nach Norm geforderten Ableitströme. Als Vorteil ist bei Ausführungsformen zu nennen, dass ein jeweiliger Verbraucher nur dann zum Ableitstrom bzw. Berührungsstrom beiträgt, wenn er aktiv ist. Nicht verwendete Verbraucher tragen nicht zum Ableitstrom bei, weil diese allpolig vom Netz getrennt werden.

### Kurze Beschreibung der Zeichnungen

Weitere Vorteile und Merkmale bevorzugter Ausführungsformen der Erfindung werden nachfolgend anhand der beiliegenden Zeichnungen erläutert, wobei die Figuren zeigen:
- Fig. 1: ist eine schematische Ansicht der Energieversorgung eines Hypothermiegeräts in Übereinstimmung mit Ausführungsformen der Erfindung; und
- Fig.2: ist ein schematisches, vereinfachtes Ablaufdiagramm von typischen Ausführungsformen erfindungsgemäßer Verfahren.

### Beschreibung bevorzugter Ausführungsbeispiele

Nachfolgend werden typische Ausführungsformen anhand der Figuren beschrieben, wobei die Erfindung nicht auf die Ausführungsbeispiele beschränkt ist, vielmehr wird der Umfang der Erfindung durch die Ansprüche bestimmt.

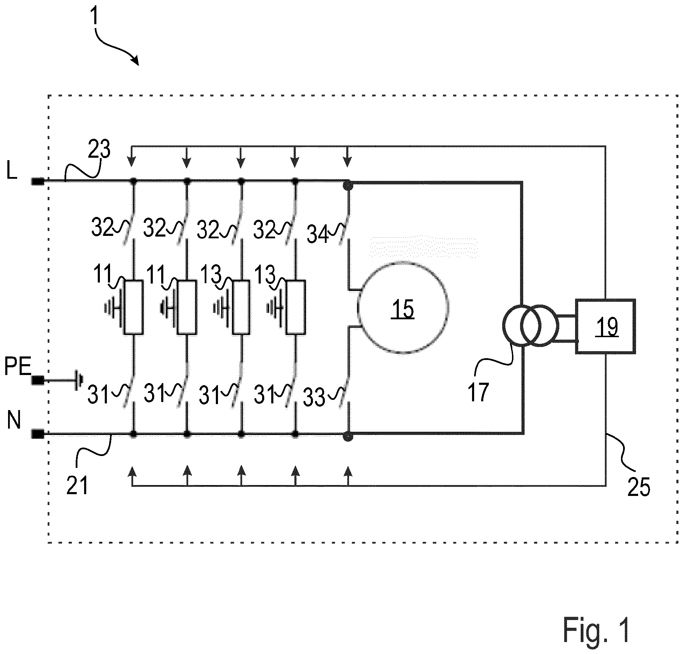

In der Fig. 1 ist die elektrische Schaltung eines erfindungsgemäßen Hypothermiegeräts 1 schematisch gezeigt.

Das beispielhafte Hypothermiegerät 1 ist für den Betrieb an einer gewöhnlichen Steckdose mit 1-Phasen Wechselspannung vorgesehen, also beispielsweise an 230 V, 50 Hz bei maximal 16 A Stromaufnahme. Weitere Ausführungsformen sind angepasst, um bei 110 V - 120 V, 60 Hz oder mit 208 - 240 V, 60 Hz betrieben zu werden, gegebenenfalls mit 12 A, 15 A, 16 A, 24 A oder mit 30 A..

Das Hypothermiegerät 1 umfasst für ein Gerät mit einer solchen Netzanschlussleistung (230 V, 16 A) vergleichsweise leistungsstarke Komponenten. Es sind zwei Hypothermie-Heizungen 11 für Hypothermie mit jeweils 1200 W Leistung entsprechend 10,4 A Gesamtstromaufnahme vorgesehen. Weiterhin sind zwei Kardioplegie-Heizungen 13 für Kardioplegie ebenfalls mit jeweils 1200 W Leistung entsprechend 10,4 A Gesamtstromaufnahme vorgesehen. Die Angaben für die Leistungsaufnahme betreffen jeweils die Leistungsaufnahme im ungetakteten Betrieb.

Weiterhin umfasst das Hypothermiegerät 1 eine Kältemaschine 15 mit einem Verdichter und einer beispielhaften Wirkleistungsaufnahme von 900 W entsprechend etwa 5,3 A einschließlich Blindanteil.

Die Hypothermie-Heizungen 11 und die Kardioplegie-Heizungen 13 werden benötigt, um beispielsweise den Patienten während des Eingriffs auf der gewünschten Temperatur zu halten oder auch beispielsweise zum Ende eines chirurgischen Eingriffs hin die Temperatur des Blutes über einen Zwischenkreis einer mit dem Hypothermiegerät 1 verbundenen Herz-Lungen-Maschine wieder aufzuheizen.

Schließlich umfasst das Hypothermiegerät 1 noch ein Netzteil 17 mit einer Leistung von 400 W entsprechend einer Stromaufnahme von ca. 1,9 A. Das Netzteil 17 dient dazu, eine Kontrolleinrichtung 19 des Hypothermiegeräts 1 einschließlich Sensoren oder ähnlicher Verbraucher mit elektrischer Energie zu versorgen. Mit der vom Netzteil 17 bezogenen elektrischen Energie werden auch weitere Verbraucher wie Kältelüfter, Pumpen und Magnetventile versorgt. Diese Verbraucher werden durch die Kontrolleinrichtung bedarfsorientiert kontrolliert.

Hierzu ist das Netzteil 17 dauerhaft während des Betriebs mit zwei Sammelschienen 21 bzw. 23 verbunden, welche eine elektrische Verbindung zu einem Nullleiter-Anschluss N bzw. zu einem Phasen-Anschluss L bereitstellen. Die Sammelschienen 21 und 23 bestehen jeweils aus Leitern, beispielsweise Kabeln, Schienen, oder Leitern einer Leiterplatte, und stellen Verteiler zum Verteilen der vom Netzanschluss bereit gestellten elektrischen Energie dar.

Zusammen stellen die Hypothermie-Heizungen 11, die Kardioplegie-Heizungen 13, die Kältemaschine 15 und das Netzteil 17 bzw. die an das Netzteil angeschlossenen Niederspannungs-Verbraucher die Verbraucher des Hypothermiegerätes 1 dar. Die Summe der Leistungen bzw. Stromaufnahmen dieser Verbraucher übersteigt mit ca. 6 kW bzw. ca. 28 A bei weitem die 3,7 kW Leistung, die aus einer mit 16 A abgesicherten Steckdose bei einer Spannung von 230 V entnommen werden kann.

Weiterhin beträgt auch ein im Fehlerfall (erster Fehler) fließender Berührungsstrom über 0,5 mA, falls alle Verbraucher mit dem Netz verbunden sind. Dies liegt daran, dass alle Verbraucher des Hypothermiegeräts 1 zum Ableitstrom beitragen. Ein zu betrachtender Fehler als erster Fehler wäre beispielsweise der Verlust der Erdung des Hypothermiegeräts 1, d.h. ein Verlust der Verbindung von einer Erdung über den Anschluss PE zum Gehäuse des Hypothermiegeräts 1. Auch bei einem ersten Fehler durch Ausfall eines der Schalter zu den Verteilern, so dass er dauerhaft geschlossen ist, bleibt der Berührungsstrom unter 0,1 mA, da dann die PE-Leitung noch funktionsfähig ist.

Das Hypothermiegerät 1 verringert den Berührungsstrom unter eine zulässige Grenze dadurch, dass bei allpoliger Trennung zumindest eines Teils der Verbraucher von den Sammelschienen 21 und 23, und nicht nur beispielsweise einpoliger Trennung nur einer Verbindung entweder zu der Sammelschiene 21 oder zu der Sammelschiene 23, erreicht werden kann, dass der Berührungsstrom reduziert wird, da die allpolig getrennten Verbraucher nicht mehr zum Berührungsstrom beitragen.

Die Kontrolleinrichtung 19 weist in der Fig. 1 nicht dargestellte Signaleingänge für Temperierungsanforderungen auf, beispielsweise kann die Kontrolleinrichtung 19 mit einer Herz-Lungen-Maschine verbunden sein, wobei die Herz-Lungen-Maschine dem Hypothermiegerät 1 über die Signaleingänge der Kontrolleinrichtung 19 Temperierungsanforderungen mitteilen kann.

Die Kontrolleinrichtung 19 steuert über einen Steuerausgang 25 die Energieversorgung der Verbraucher. So kann die Kontrolleinrichtung mittels jeweils zwei als TRIAC ausgebildeten Schaltern 31 und 32 jede der Kardioplegie-Heizungen 13 und jede der Hypothermie-Heizungen 11 allpolig von den Sammelschienen 21 und 23 trennen. Ein TRIAC kann also jeweils einen Schalter 31 und einen Schalter 32 umfassen, es können jedoch auch getrennte Komponenten für die Schalter 31 einerseits und die Schalter 32 andererseits vorgesehen sein. Dabei schalten die Schalter 31 jeweils die Verbindung zwischen Kardioplegie-Heizung 13 bzw. der Hypothermie-Heizung 11 und der mit N verbundenen Sammelschiene 21. Und die Schalter 32 schalten jeweils die Verbindung zwischen Kardioplegie-Heizung 13 bzw. der Hypothermie-Heizung 11 und der mit L verbundenen Sammelschiene 23.

Weiterhin kann die Steuereinrichtung 19 über zwei als Relais ausgebildete einpolige Schalter 33 und 34 die Kältemaschine 15 allpolig von den Sammelschienen 21 und 23 trennen oder mit ihnen verbinden. Die Schalter 33 und 34 können dabei in einem Relais, welches beide Pole schaltet, vereint sein.

Die als TRIAC ausgeführten Schalter 31 und 32 werden durch die Kontrolleinrichtung 19 mittels einer Halbwellenansteuerung angesteuert. Mit der Halbwellensteuerung kann die maximale Heizleistung der Heizungen 11 und 13 jeweils in Netzhalbwellen aufgeteilt werden.

Die Kältemaschine 15 wird durch die Kontrolleinrichtung 19 über die als Relais ausgebildeten Schalter 33 und 34 minutenweise zu- und abgeschaltet.

Es können Leistungsmanagement-Verfahren, welche dynamisch und prioritätsorientiert nachrangige Heizungen abregeln, sodass die Gesamtstromaufnahme nie über 16 A liegt, verwendet werden. Durch die allpolige Trennung bei Abregelung kann erreicht werden, dass auch die Grenzen für den Ableitstrom bzw. Berührungsstrom eingehalten werden.

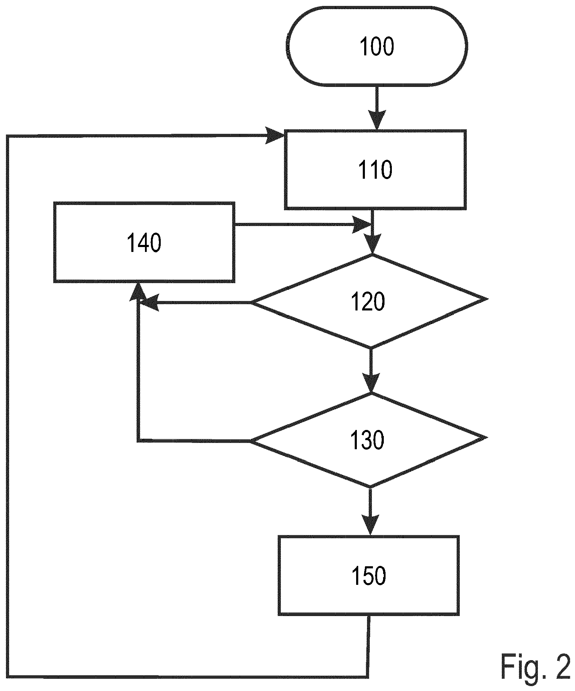

In der Fig. 2 ist ein typisches erfindungsgemäßes Verfahren gezeigt. Das Verfahren der Fig. 2 kann mit einem Hypothermiegerät 1 entsprechend der Fig. 1 ausgeführt werden. Gleiche Teile wie in der Fig. 1 werden im Zusammenhang mit der Fig. 2 nicht nochmals erläutert.

Das Verfahren zum Steuern eines Hypothermigerätes 1 wie in Fig. 2 dargestellt startet in einem Block 100.

Im Block 110 werden nachfolgend bestimmte Verbraucher in Abhängigkeit von Betriebsparametern des Hypothermiegeräts und/oder in Abhängigkeit einer über einen Signaleingang empfangenen Temperierungsanforderung ausgewählt. Dies kann beispielsweise eine Auswahl von Heizungen oder eine Auswahl der Kältemaschine entsprechend einer Temperierungs-Vorgabe durch eine Herz-Lungen-Maschine sein.

Im Block 120 wird überprüft, ob bei Verbinden der ausgewählten Verbraucher mit den Sammelschienen eine erste zuvor festgelegte Anforderung erfüllt bleibt. Diese erste Anforderung betrifft einen Grenzwert für eine maximale Stromaufnahme. Diese muss bei maximal 16 A liegen. Diese Überprüfung kann an Hand von in einer Tabelle mit für jeden Verbraucher hinterlegten Stromaufnahmen vorgenommen werden.

Im Block 130 wird überprüft, ob bei Verbinden der ausgewählten Verbraucher mit den Sammelschienen eine zweite zuvor festgelegte Anforderung erfüllt bleibt. Diese Überprüfung kann an Hand von in einer Tabelle mit für jeden Verbraucher hinterlegten Ableitströmen vorgenommen werden. Diese zweite Anforderung betrifft einen Grenzwert für den maximalen Ableitstrom im Falle eines so genannten ersten Fehlers. Der maximale Ableitstrom darf im Falle eines ersten Fehlers 0,5 mA nicht überschreiten.

Bei weiteren Ausführungsformen werden wird nur die Stromaufnahme überprüft, das heißt der Block 130 entfällt ersatzlos. Die Überprüfung in Block 120, also die Überprüfung der maximalen Stromaufnahme bewirkt indirekt die Einhaltung des Grenzwerts des Ableitstroms. Typischerweise wird die Einhaltung der Grenzwerte für Erdableitstrom und Berührungsstrom bei typischen Ausführungsformen bei der Endprüfung in der Fertigung sowie bei der jährlichen sicherheitstechnischen Inspektion geprüft.

Ergibt eine der Überprüfungen der Blöcke 120 und 130, dass eine der beiden Anforderungen maximale Stromaufnahme von 16 A und maximaler Ableitstrom im ersten Fehlerfall von maximal 0,5 mA nicht erfüllt wird, springt das Verfahren jeweils zu einem Block 140.

Im Block 140 wird mindestens ein Verbraucher, welcher zuvor im Schritt 110 ausgewählt wurden, an Hand eines Prioritätsschemas abgewählt. Dieses Abwählen bedeutet, dass der mindestens eine abgewählte Verbraucher nicht mehr zu den ausgewählten Verbrauchern zählt und dementsprechend nicht mehr dazu vorgesehen ist, mit den beiden Verteilern verbunden zu werden bzw. falls er bereits verbunden ist, dazu vorgesehen ist, von den Verteilern getrennt zu werden. "Abwählen" im Block 140 umfasst dabei typischerweise auch eine Leistungsreduktion von abgewählten Verbrauchern durch eine getaktete Halbwellenansteuerung. Dabei wird der jeweilige Verbraucher, typischerweise eine der Heizungen, durch kurzfristiges, getaktetes und allpoliges Trennen und Verbinden in der Leistung reduziert, wobei auch der Beitrag zum Ableitstrom des Hypothermiegerätes reduziert wird.

Anschließend durchläuft das Verfahren wieder die Blöcke 120 und 130, um zu prüfen, ob die Anforderungen mit den verbleibenden ausgewählten Verbrauchern erfüllt wird.

Ergibt die Überprüfung, dass beide Anforderungen eingehalten werden, läuft das Verfahren mit dem Block 150 weiter, in welchem die Schalter entsprechend der ausgewählten Verbraucher und ggf. mit der entsprechenden Halbwellenansteuerung mit elektrischer Energie versorgt werden. Sollte in Block 140 eine Reduzierung beispielsweise einer Kühl- oder Heizleistung erfolgt sein, so kann es zu einer Verlängerung von Abkühl- oder Aufheizzeiten während der OP kommen. Demgegenüber steht allerdings die zuverlässige Einhaltung von Grenzwerten wie oben beschrieben.

Nachfolgend beginnt das Verfahren mit dem Block 110 von vorne. Dabei kann eine Taktzeit von 2 s vorgesehen sein. Weiterhin ist vorgesehen, dass die Kältemaschine als Verbraucher frühestens nach einer Minute wieder in einen anderen Zustand, also getrennt oder verbunden mit den Sammelschienen, versetzt werden darf.

## Patentansprüche

1. Hypothermiegerät mit
einer Mehrzahl von Verbrauchern, welche im Betrieb elektrische Energie verbrauchen;
einem Netzanschluss zur Versorgung der Verbraucher mit elektrischer Energie über zumindest zwei Verteiler zum Verteilen der elektrischen Energie ; und
einer Mehrzahl von Schaltern, wobei mindestens einer der Verbraucher mittels mindestens einem ihm zugeordneten Schalter (31, 32, 33, 34) mit den Verteilern verbindbar ist;
wobei die Schalter (31, 32, 33, 34) derart ausgeführt sind, dass die Verbraucher allpolig gegenüber den Verteilern schaltbar sind.

2. Hypothermiegerät (1) nach Anspruch 1, mit einer Kontrolleinrichtung, welche dazu eingerichtet ist, in einem Patienten-Betrieb zumindest einen der Verbraucher mittels des diesem Verbraucher zugeordneten mindestens eines Schalters (31, 32, 33, 34) allpolig von den Verteilern zu trennen.

3. Hypothermiegerät (1) nach Anspruch 2, wobei der Patienten-Betrieb einen Betrieb mit Temperierung eines Patienten umfasst

4. Hypothermiegerät (1) nach einem der Ansprüche 2 oder 3, wobei die Kontrolleinrichtung so viele der Verbraucher mittels der diesen Verbrauchern jeweils zugeordneten Schaltern (31, 32, 33, 34) allpolig von den Verteilern trennt, so dass in einem fehlerfreien Zustand des Hypothermiegeräts ein Erdableitstrom von 0,1 mA und/oder im Fall eines ersten Fehlers ein Berührungsstrom von 0,5 mA nicht überschritten wird.

5. Hypothermiegerät (1) nach einem der vorherigen Ansprüche, wobei die Verbraucher zumindest zwei der folgenden Einrichtungen umfassen: eine Hypothermie-Heizung (11), eine Kardioplegie-Heizung (13), eine Kältemaschine (15) und ein Netzteil (17).

6. Hypothermiegerät (1) nach einem der vorhergehenden Ansprüche, mit einer Kontrolleinrichtung, welche einen Signaleingang umfasst und welche dazu eingerichtet ist:
- Auswählen bestimmter Verbraucher in Abhängigkeit von Betriebsparametern des Hypothermiegeräts und/oder in Abhängigkeit einer über den Signaleingang empfangenen Temperierungsanforderung;
- Überprüfen, ob bei Verbinden der ausgewählten Verbraucher zumindest eine zuvor festgelegte Anforderung erfüllt bleibt; und
- Verbinden der ausgewählten Verbraucher mittels der diesen Verbrauchern zugeordneten Schaltern (31, 32, 33, 34) mit den Verteilern und/oder allpoliges Trennen der anderen Verbraucher von den Verteilern mittels der diesen anderen Verbrauchern zugeordneten Schaltern (31, 32, 33, 34).

7. Hypothermiegerät (1) nach Anspruch 6, wobei die Kontrolleinrichtung weiterhin eingerichtet ist, so dass der Schritt des Überprüfens umfasst:
- Falls die mindestens eine zuvor festgelegte Anforderung mit den zuvor ausgewählten Verbrauchern nicht erfüllt bleibt: Abwählen von bestimmten in dem Schritt des Auswählens ausgewählter Verbraucher an Hand eines abgespeicherten Prioritätsschemas, so dass die zuvor festgelegte mindestens eine Anforderung mit den verbleibenden ausgewählten Verbrauchern erfüllt wird.

8. Hypothermiegerät (1) nach einem der Ansprüche 6 oder 7, wobei die Anforderung ein Grenzwert für den maximalen Ableitstrom ist des Hypothermiegerätes (1) ist und/oder ein Grenzwert für eine maximale Stromaufnahme der Verbraucher über die Verteiler.

9. Hypothermiegerät (1) nach einem der vorhergehenden Ansprüche, wobei die Schalter (31, 32, 33, 34) elektronische Schalter (31, 32) und/oder Relais (33, 34) umfassen.

10. Hypothermiegerät (1) nach Anspruch 9, wobei als elektronische Schalter (31, 32) TRIAC-Halbleiter verwendet werden.

11. Hypothermiegerät (1) nach einem der vorhergehenden Ansprüche, wobei zumindest einige der Schalter (31, 32) mittels einer Halbwellenansteuerung angesteuert werden.

12. Verfahren zum Steuern eines Hypothermiegerätes (1) nach einem der vorhergehenden Ansprüche mit:
- Auswählen bestimmter Verbraucher in Abhängigkeit von Betriebsparametern des Hypothermiegeräts (1) und/oder in Abhängigkeit einer über einen Signaleingang empfangenen Temperierungsanforderung;
- Überprüfen, ob bei Verbinden der ausgewählten Verbraucher zumindest eine zuvor festgelegte Anforderung erfüllt bleibt; und
- Verbinden der ausgewählten Verbraucher mittels der diesen Verbrauchern zugeordneten Schaltern mit den Verteilern und/oder allpoliges Trennen der anderen Verbraucher von den Verteilern mittels der diesen anderen Verbrauchern zugeordneten Schaltern (31, 32, 33, 34).

13. Verfahren nach Anspruch 12, wobei der Schritt des Überprüfens umfasst:
- Falls die zuvor festgelegte Anforderung mit den zuvor ausgewählten Verbrauchern nicht erfüllt bleibt: Abwählen von bestimmten in dem Schritt des Auswählens ausgewählter Verbraucher an Hand eines abgespeicherten Prioritätsschemas, so dass die zuvor festgelegte Anforderung mit den verbleibenden ausgewählten Verbrauchern erfüllt wird.
